Europäisches Patentamt

European Patent Office

Office européen des brevets

(19)

(11) Veröffentlichungsnummer: **0 113 085**
A1

(12) # EUROPÄISCHE PATENTANMELDUNG

(21) Anmeldenummer: 83112611.5

(22) Anmeldetag: 15.12.83

(51) Int. Cl.³: **C 08 K 5/20**
A 61 K 7/46, A 61 L 9/04

(30) Priorität: 23.12.82 DE 3247708

(43) Veröffentlichungstag der Anmeldung:
11.07.84 Patentblatt 84/28

(84) Benannte Vertragsstaaten:
AT BE CH DE FR GB IT LI LU NL SE

(71) Anmelder: Henkel Kommanditgesellschaft auf Aktien
Postfach 1100 Henkelstrasse 67
D-4000 Düsseldorf-Holthausen(DE)

(72) Erfinder: Schumann, Klaus, Dr.
Keplerstrasse 33
D-4006 Erkrath(DE)

(72) Erfinder: Kittscher, Peter
Edelweisstrasse 25
D-4044 Kaarst 2(DE)

(72) Erfinder: Altenschöpfer, Theodor, Dr.
Einsteinstrasse 3
D-4000 Düsseldorf 13(DE)

(72) Erfinder: Streschnak, Benno, Dr.
Schönwasserstrasse 259
D-4015 Krefeld(DE)

(54) Mittel und dessen Verwendung zum Beduften von Kunststoffmaterialien.

(57) Zum mechanisch stabilen Beduften von nicht-saugfähigen Kunststoffmaterialien, insbesondere Innenflächen von Verpackungskartons für Wasch- und Reinigungsmittel dienen wäßrige und/oder lösungsmittelhaltige Lösungen oder Suspensionen, die im wesentlichen Duftstoffe und filmbildende, geruchslose, organische Polymere, insbesondere Polyvinylacetat oder vinylacetathaltige Copolymere enthalten. Das Auftragen der Suspension zur klebfreien Duftfilmbildung auf Kunststoffmaterialien erfolgt durch Sprühen, Streichen oder Rakeln.

EP 0 113 085 A1

0113085

4000 Düsseldorf, den 21.12.1982
Henkelstraße 67

HENKEL KGaA
ZR-FE/Patente
Dr. Wf/Ne

Patentanmeldung
D 6721 EP

Mittel und dessen Verwendung zum Beduften von Kunststoffmaterialien

Die Erfindung betrifft ein Mittel bzw. dessen Verwendung
zum Beduften von nicht-saugfähigen Kunststoffmaterialien,
insbesondere von Innenräumen von daraus hergestellten
Verpackungsbehältern, vor allem für Wasch- und Reinigungsmittel.

Wasch- und Reinigungsmittel werden üblicherweise in parfümierter Form angeboten. Hierbei sind die Duftstoffe in
der Regel unmittelbar im Produkt enthalten, so daß der
Verbraucher das Dufterlebnis sowohl beim Öffnen der Verpackung als auch bei Anwendung der Produkte hat.

Duftstoffe sind jedoch gegenüber einer Vielzahl von Inhaltsstoffen von Wasch- und Reinigungsmitteln instabil.
Das gilt z. B. für Chlor als Hypochlorit oder gebunden an
organische Chlorträger, wie Natriumdichlorisocyanurat und
Trichlorisocyanursäure. Auch Perborat in Kombination mit
Bleichaktivatoren kann die Stabilität von Duftstoffen negativ beeinflussen. Diese Nachteile können gemäß der
EP 0 004 463 A 2 durch Beduften des üblicherweise saugfähigen Verpackungsmaterials selbst, also insbesondere der
überwiegend als Verpackung verwendeten Kartons, vermieden
werden. Ein direkter Kontakt der Duftstoffe mit den störenden Inhaltsstoffen findet dann praktisch nicht statt.
Vorzugsweise werden die Duftstoffe im inneren oberen Teil
der Verpackung mit dem Ziel eines Imprägnierens des Kartonmaterials aufgetragen.

In der Praxis hat sich aber gezeigt, daß viele Kartonmaterialien die durch Tropfen oder Sprühen aufgebrachten
Duftstoffe nur langsam in sich aufnehmen. Die Duftstoffe

...

verlaufen daher "nasenförmig" und es entstehen sichtbare
Flecken. Ferner treten beim Versprühen der Duftstoffe und
der damit verbundenen Duftstoffvernebelung erhebliche,
mit Geruchsbelästigung der Umgebung verbundene Verluste
auf. Daher wird in der älteren deutschen Patentanmeldung
P 32 01 941.6 ein Verfahren zum Beduften von Verpackungsmaterialien vorgeschlagen, bei dem der Duftstoff vor dem
Auftragen in eine höherviskose Paste eingearbeitet und in
Form dieser Paste auf den inneren, insbesondere üblicherweise nicht befüllten Teil des Verpackungsmaterials aufgetragen wird. Hierbei wird also wiederum auf eine direkte Parfümierung der Produkte verzichtet und dadurch der
Duftstoffverbrauch erheblich beschränkt. Ferner wird
durch die Verwendung des Duftstoffs in Pastenform eine
produktionstechnisch praktikable Lösung unter spezieller
Berücksichtigung der verminderten Duftstoffbelastung am
Arbeitplatz sowie verringerter Duftstoffverluste ermöglicht.

In diesen bekannten Fällen werden Verpackungs- und Trägermaterialien beschrieben, die mit Duftstoffen oder pastenförmigen Zubereitungen der Duftstoffe mittels Sprühen oder Bestreichen "imprägniert" werden. Es handelt
sich somit um saugfähige Materialien, die mehr oder weniger große Anteile der Duftstoffe aufnehmen.

Die Saugfähigkeit der Verpackungs- und Trägermaterialien
ist Voraussetzung für die Realisierung der Parfümierungsmethode, da andernfalls das Verlaufen dieser Dufstoffe
oder der direkte Kontakt der Haut mit konzentrierten Parfümölen bei der Entnahme von Produkten aus den Verpackungen nicht auszuschließen ist, was bei empfindlichen Personen zu Irritationen der mit Parfümöl kontaktierten Haut
führen kann.

...

Es gibt aber auch nicht saugfähige Verpackungs- und Trägermaterialien, beispielsweise aus Kunststoffen oder kunststofflaminierten Pappen. Dafür sind die bekannten Beduftungsmöglichkeiten nicht geeignet.

Aus der GE-PS 1 515 931 ist bereits bekannt, daß man Flächen aus verschiedenen thermoplastischen Kunststoffen, wie beispielsweise von Plastikbeuteln oder -säcken, mit einer schnelltrocknenden flüssigen Mischung aus einem Duftstoff und einem Trägermaterial beschichten kann, um ihnen einen für den Verbraucher angenehmen Geruch zu vermitteln. Als Trägermaterial werden beispielsweise Nitrocellulose, Schellack und Polyvinylbutyral genannt. Die Beschichtung kann unter anderem durch Aufsprühen der Mischung auf die Kunststoffoberflächen erfolgen.

Nach einem Verfahren gemäß der japanischen Patentanmeldung 73 22 132 können Kunststoffregale durch Überziehen mit einer Emulsion aus Holzduft, Polyvinylalkohol und Kornstärke der Vorstellung von echten Holzregalen angenähert werden.

Demgegenüber war es aber überraschend, daß man eine wäßrige, gegebenenfalls lösungsmittelhaltige Lösung oder Suspension aus einem Duftstoff und einem organischen, vorzugsweise vinylacetathaltigen Polymeren bzw. Mischpolymeren sowie gegebenenfalls einem Weichmacher zur klebfreien, mechanisch stabilen Duftfilmbildung auf Kunststoffmaterialien, insbesondere von Verpackungsteilen für Wasch- und Reinigungsmittel, verwenden kann.

Die Erfindung betrifft daher ein Mittel, das im wesentlichen aus wäßrigen und/oder lösungsmittelhaltigen Lösungen oder Suspensionen von Duftstoffen und filmbildenden, geruchsfreien, organischen Polymeren besteht und dessen

...

Verwendung zum klebfreien, mechanisch stabilen Beduften von nicht-saugfähigen Kunststoffmaterialien, insbesondere von Innenräumen von daraus hergestellten oder damit überzogenen Verpackungsbehältern durch Aufsprühen, Aufstreichen oder Aufrakeln.

Geeignete Polymere sind Polyvinylacetat, Mischpolymere aus Vinylacetat und Maleinsäure-n-butylester sowie Vinylacetat und Crotonsäure, ebenso wie Carboxymethylcellulose oder Polyacrylate. Die Polymeren können allein oder in Kombination miteinander verwendet werden. Ihre Menge in der Flüssigkeit oder Paste liegt bei 20 bis 50, vorzugsweise bis 40 Gew.-%.

Die Lösungen bzw. Suspensionen der Polymeren können noch geringe Mengen (weniger als 1 Gew.-%) an Weichmachern wie z. B. Dibutylphthalaten oder Trikresylorthophosphaten, sowie Füllstoffen (Pigmenten), wie z. B. Montmorillonite, Bentonite oder hochdisperse Kieselsäure (Aerosil® ), enthalten. Hierdurch werden die Viskosität der Lösungen bzw. Suspensionen sowie die Eigenschaften der filmartigen Überzüge beeinflußt. Die Menge an Füllstoffen kann bis zu 5 Gew.-% betragen.

Die anwendungstechnisch günstigsten filmartigen, die Duftstoffe enthaltenden Überzüge werden aus Polymerensuspensionen oder -lösungen gebildet, deren Einfriertemperatur (Weißpunkt) zwischen 10 bis 20 °C liegt. Bevorzugt werden wäßrige Systeme, da diese aus verfahrenstechnischen Gründen Vorteile gegenüber solchen mit organischen Lösungsmitteln besitzen. Die Viskosität der Polymerensuspensionen bzw. -lösungen liegt zwischen 500 bis 5000, vorzugsweise zwischen 1000 und 2500 m Pa·s. Sie wird mit einem Rotationsviskosimeter bei 20 °C gemessen. Der pH-Wert der Systeme liegt zwischen 3,5 und 7, vorzugsweise zwischen 4 und 5,5.

...

In die Polymerensuspensionen können verschiedenste Parfümöle in einer Konzentration von 0,1 - 50, vorzugsweise 10 bis 30 Gew.-% eingearbeitet werden. Mit höherem Parfümölgehalt kann ständiges Rühren der duftstoffhaltigen Suspensionen erforderlich werden, um das Ausrahmen der Parfümöle zu verhindern. Durch den Zusatz von Pigmenten, wie Kieselsäure-Aerosil®, kann die Abtrennung der Parfümöle verhindert und gleichzeitig die Viskosität der Polymerensuspensionen erhöht werden. Die Einarbeitung der Parfümöle und Pigmente erfolgt vorteilhaft mittels Rührwerken, die vorzugsweise hohe Scherkräfte ausüben.

Die Endviskosität der dabei entstehenden Duftstoffpasten hängt ab vom Verhältnis der Menge an Duftstoff zur Pigmentmenge. Zum weiteren Beeinflussen der Viskosität können den Duftstoffen Lösungsmittel mit unterschiedlicher Polarität zugesetzt werden. Der Begriff "Duftstoff" umfaßt im Rahmen der Erfindung sowohl einzelne Duftstoffe als auch Duftstoffkombinationen. Ähnliches gilt für den Begriff "Pigmente".

Geeignete Lösungsmittel für die Polymeren sind außer Wasser organische Lösungsmittel, wie z. B. niedermolekulare Alkohole, die bei Raumtemperatur verdunsten und mit einem eventuell verbleibenden Restgehalt die Eigenschaften des Polymerenfilms nicht negativ beeinflussen.

Als Verpackungsformen sind Rund-, Rechtecktrommeln und Pakete jeder Größe, beispielsweise aus Polyethylen, Polypropylen, Polyvinylchlorid, Polystyrol oder Polyurethan, geeignet. Der Auftrag der duftstoffhaltigen Polymerenlösungen bzw. -suspensionen oder -pasten erfolgt im oberen Drittel der Verpackungen, bevorzugt im oberen freien Leerraum. Bei Tommelverpackungen kann auch das Deckmaterial, also gegebenenfalls ein Kunststoffdeckel, als Träger für die duftstoffhaltigen Suspensionen verwendet wer-

...

den. In diesem Fall kann die Verpackung selbst auch aus saugfähigen Materialien bestehen, die dann jedoch ihrerseits nicht beduftet zu werden brauchen.

Das Aufbringen der duftstoffhaltigen Polymerensuspensionen, -lösungen oder -pasten auf die Verpackungs- oder Trägermaterialien aus Kunststoff oder mit Kunststoffüberzügen erfolgt durch Sprühen, Streichen oder Rakeln.

Besondere Vorteile der neuen Mittel sind Duftstoffstabilität in Gegenwart alkalischer und saurer Rohstoffe, Oxidationsmittel, wie z. B. organische Chlorträger oder Peroxyverbindungen sowie die gleichmäßige Abgabe der Duftstoffe im Verlauf auch einer längeren Lagerzeit.

<u>Beispiel</u>

Eine Duftstoffpaste, bestehend aus

50 Gew.-% einer Suspension aus 37 - 42 Gew.-% Polyvinylacetat (Mowilith® der Fa. Hoechst) und weniger als 1 Gew.-% Dibutylphthalat mit einer Viskosität von 1500 - 2000 mPa·s (gemessen mit einem Rotationsviskosimeter bei 20 °C) und einem pH-Wert von 4 - 5,

46,25 Gew.-% Parfümöl (Duftnote "grüner Apfel",

3,75 Gew.-% feinteilige Kieselsäure (Aerosil® der Fa. Degussa),

wurde in einer Menge von 1,1 g mittels einer Düse mit kegelförmigem Austrittsbild bei einem Druck von ca. 1 - 3 bar auf die Innenfläche eines Kunststoffdeckels (Polypropylen) mit Ø 225 mm aufgedüst. Die Verteilung erfolgte möglichst gleichmäßig über die Fläche.

Nach ca. 1 Stunde war die Deckelfläche grifftrocken. Es bildete sich ein duftstoffhaltiger, mechanisch stabiler Film. Im Verlauf des 12wöchigen Lagertests, bei dem die Trommel, die zu 4/5 mit einem alkalischen, chlorträgerhaltigen Reinigungsmittel gefüllt war, täglich einmal 3 Minuten lang geöffnet wurde, war die Duftintensität deutlich höher als beim Aufsprühen des reinen Parfümöles. Die Innenfläche des Deckels blieb trocken und führte zu keinerlei Beanstandung durch die Testpersonen.

...

0113085

HENKEL KGaA
ZR-FE/Patente

P A T E N T A N S P R Ü C H E

1. Mittel zum Beduften von nicht-saugfähigen Kunststoffmaterialien, insbesondere von Innenräumen von daraus hergestellten oder damit überzogenen Verpackungsbehältern, dadurch gekennzeichnet, daß sie im wesentlichen aus wäßrigen und/oder lösungsmittelhaltigen Lösungen oder Suspensionen von Duftstoffen und filmbildenden, geruchsfreien, organischen Polymeren bestehen.

2. Mittel nach Anspruch 1, dadurch gekennzeichnet, daß die organischen Polymeren aus Polyvinylacetat bestehen oder vinylacethaltige Copolymerisate sind.

3. Mittel nach Anspruch 1 und 2, dadurch gekennzeichnet, daß sie einen Polymerengehalt von 20 bis 50, vorzugsweise bis 40 Gewichtsprozent aufweisen.

4. Mittel nach Anspruch 1 bis 3, dadurch gekennzeichnet, daß sie eine Duftstoffkonzentration von 0,1 bis 50, vorzugsweise von 10 bis 30 Gewichtsprozent aufweisen.

5. Mittel nach Anspruch 1 bis 4, dadurch gekennzeichnet, daß sie zusätzlich weniger als 1 Gewichtsprozent, bezogen auf das Polymere, an einem Weichmacher enthalten.

6. Mittel nach Anspruch 1 bis 5, dadurch gekennzeichnet, daß sie zusätzlich bis zu 5 Gewichtsprozent, bezogen auf das Mittel, an einem Füllstoff enthalten.

7. Mittel nach Anspruch 1 bis 6, dadurch gekennzeichnet, daß sie einen Weißpunkt zwischen 10 und 20 °C aufweisen.

8. Mittel nach Anspruch 1 bis 7, dadurch gekennzeichnet, daß sie eine Viskosität von 500 - 5000, vorzugsweise von 1000 - 2500 mPa·s bei 20 °C aufweisen.

9. Mittel nach Anspruch 1 bis 8, dadurch gekennzeichnet, daß sie einen pH-Wert zwischen 3,5 und 7, vorzugsweise zwischen 4 und 5,5 aufweisen.

10. Verwendung eines Mittels, bestehend im wesentlichen aus wäßrigen und/oder lösungsmittelhaltigen Lösungen oder Suspensionen von Duftstoffen und filmbildenden, geruchsfreien organischen Polymeren zum klebfreien mechanisch stabilen Beduften von nicht-saugfähigen Kunststoffmaterialien.

11. Verwendung eines Mittels nach Anspruch 10 zum Beduften von Innenräumen von aus Kunststoffen hergestellten oder damit überzogenen Verpackungsmaterialien, insbesondere für Wasch- und Reinigungsmittel.

12. Verwendung eines Mittels nach Anspruch 10 und 11, bestehend im wesentlichen aus einem Duftstoff und Polyvinylacetat oder einem vinylacetathaltigen Copolymeren.

13. Verwendung eines Mittels nach Anspruch 10 bis 12 durch Sprühen, Streichen oder Rakeln.

. . .

**Europäisches Patentamt**

## EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| X | US-A-4 209 417  (D. WHYTE)<br>* Beispiele 1,3 * | 1,3-9 | C 08 K   5/00<br>A 61 K   7/46<br>A 61 L   9/04 |
| X | DD-A- 115 693  (A. KÄMMER)<br>* Ansprüche; Beispiel * | 1,3-13 | |
| X | FR-A-1 176 992  (H. GAMARD)<br>* Insgesamt * | 1-13 | |
| X | CHEMICAL ABSTRACTS, Band 93, Nr. 20, November 1980, Seite 351, Nr. 191919j, Columbus, Ohio, US<br>& JP - A - 80 85 515 (LION CORP.) 27-06-1980 | 1-13 | |
| X | US-A-3 926 655  (J. MILES)<br>* Ansprüche 1,4 * | 10-13 | RECHERCHIERTE SACHGEBIETE (Int. Cl. ³) |
| X | US-A-4 095 031  (E. ENGLE)<br>*  Ansprüche;  Spalte  2,  Zeilen 14-18 * | 1-13 | C 08 K<br>C 08 L<br>A 61 K<br>A 61 L |

Der vorliegende Recherchenbericht wurde für alle Patentansprüche erstellt.

| Recherchenort<br>DEN HAAG | Abschlußdatum der Recherche<br>16-03-1984 | Prüfer<br>HOFFMANN K.W. |
|---|---|---|

**KATEGORIE DER GENANNTEN DOKUMENTEN**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

 

& : Mitglied der gleichen Patentfamilie, übereinstimmendes Dokument

EPA Form 1503 03.82